Europäisches Patentamt

European Patent Office

Office européen des brevets

(11) Publication number: **0 107 274 B1**

(12) **EUROPEAN PATENT SPECIFICATION**

(45) Date of publication of patent specification: **05.03.86**

(51) Int. Cl.⁴: **B 01 J 27/18,** C 07 C 51/215, C 07 C 51/25, C 07 C 57/145

(21) Application number: **83304174.2**

(22) Date of filing: **19.07.83**

(54) **Attrition resistant microspheroidal fluid bed catalysts containing the mixed oxides of vanadium and phosphorus.**

(30) Priority: **23.09.82 US 422120**

(43) Date of publication of application:
**02.05.84 Bulletin 84/18**

(45) Publication of the grant of the patent:
**05.03.86 Bulletin 86/10**

(84) Designated Contracting States:
**BE DE FR GB IT NL**

(56) References cited:
**EP-A-0 056 901**
**FR-A-2 022 271**

(73) Proprietor: **THE STANDARD OIL COMPANY**
**Midland Building**
**Cleveland, Ohio 44115 (US)**

(72) Inventor: **Bremer, Noel Jerome**
**3633 By Lane**
**Kent Ohio 44240 (US)**
Inventor: **Milberger, Ernest Carl**
**34765 Sherwood Drive**
**Solon Ohio 44139 (US)**
Inventor: **Dria, Dennis Edward**
**3604 Ambleside Drive**
**Austin Texas 78759 (US)**
Inventor: **Nicholas, Mark Lee**
**2666 N. Moreland**
**Cleveland Ohio 44120 (US)**
Inventor: **Blum, Patricia Rae**
**970 Brookpoint Drive**
**Macedonia Ohio 44056 (US)**

(74) Representative: **Smith, Sydney et al**
**Elkington and Fife High Holborn House 52/54**
**High Holborn**
**London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

# 0 107 274

**Description**

This invention relates to a method for preparing attrition resistant fluid bed catalysts useful in the production of dicarboxylic acid anhydrides by the oxidation of hydrocarbons. More particularly it is directed to the preparation of fluid bed catalysts suitable for producing maleic anhydride from 4-carbon atom hydrocarbons, such as n-butane, n-butenes, 1,3-butadiene or a mixture thereof.

The advantages of fluid bed hydrocarbon oxidation processes compared to fixed bed hydrocarbon oxidation processes are well known in the art, including the improvement of temperature control and heat transfer for oxidation reactions. Catalysts which are suitable for fixed bed processes, however, are not necessarily suitable for fluid bed processes. Catalysts which are suitable for fixed bed processes in which there are few attriting forces in operation may be too "soft" to withstand the attrition caused by fluid bed operation.

Attrition of the catalyst in fluid bed operations, that is the removal of the outer layer of the catalyst particle by abrasion or the breakup or fracture of the catalyst particle is caused by impact of the fluidized particles with each other, with the walls of the fluid bed vessel, especially within the reactor cyclones which trap the fluidized catalyst particles before they escape from the reactor to return to the catalyst bed.

Catalysts containing vanadium and phosphorus oxides have been used in the oxidation of 4-carbon atom hydrocarbons, such as n-butane, n-butenes, 1,3-butadiene or mixtures thereof with molecular oxygen or oxygen-containing gas to produce maleic anhydride. Conventional methods of preparing these catalysts involve reducing a pentavalent vanadium compound, and combining the same with a phosphorus compound, and if desired, promoter element compounds under conditions which will provide vanadium in a valence state below +5 to form catalyst precursors capable of being converted to an oxide. The catalyst oxide precursor is then recovered and calcined, before or after the fixed bed catalyst particles are formed, to provide active catalytic material.

U.S. Patents, 3,888,886; 3,905,914; 3,931,046; 3,932,305 and 3,975,300 disclose the testing of promoted vanadium phosphorus oxide catalysts for maleic anhydride production from butane in one inch (2.54 cm) diameter fluid bed reactors. In most instances, the catalysts were prepared by forming the catalyst precursor in aqueous media (in 3,975,300 the precursor was formed in a paste of a vanadium compound, a phosphorus compound and an organic reducing agent), drying and thereafter grinding and sieving the precursor to a powder of about 74 to 250 micrometers size. This manner of preparation, however, does not yield the attrition resistant catalyst particles preferred for successful fluid bed operation. Therefore, this problem remains to be solved.

EP—A—56901 describes a process for the preparation of a fluid bed catalyst containing the mixed oxydes of vanadium and phosphorus comprising

(a) preparing a vanadium phosphorus mixed oxide containing catalyst precursor;

(b) comminuting the catalyst precursor, preferably to an average particle size less than one micron in diameter;

(c) introducing the catalyst precursor into water prior or subsequent to said comminuting to form an aqueous slurry and spray drying said slurry to form microspheroidal catalyst particles.

The comminuting step may be accomplished by dry or wet ball milling.

Commercial fluid bed catalysts are preferably microspheroidal particles within the range of 20 to 300 microns in average diameter, preferably having 80% of the particles within the range of 30 to 80 microns in diameter. Most preferably, 25 to 40% of the particles have an average diameter of less than 45 microns.

It is an object of the invention to provide a process of preparing attrition resistant fluid bed vanadium and phosphorus mixed oxide-containing oxidation catalysts whereby the above mentioned problem is at least in part solved.

It is a further object of the invention to provide a process for producing maleic anhydride from 4-carbon atom hydrocarbons utilizing attrition resistant fluid bed vanadium phosphorus mixed oxide catalysts.

We have found according to the invention that excellent uniform, attrition resistant microspheroidal fluid bed catalysts, containing the mixed oxides of vanadium and phosphorus, useful in the production of maleic anhydride from 4-carbon atom hydrocarbons can be obtained by the process which includes the steps of:

(a) preparing a vanadium-phosphorus mixed oxide containing catalyst precursor;

(b) densifying the catalyst precursor;

(c) comminuting the catalyst precursor to an average particle size less than one micrometer in diameter;

(d) forming microspheroidal fluidizable particles from the densified, comminuted catalyst precursor; and

(e) calcining the microspheroidal fluidizable particles under fluidization-type conditions.

The present invention provides the attrition resistant fluid bed catalysts prepared by the above process, and further provides a process for utilizing such attrition resistant catalysts in the production of maleic anhydride in the vapor phase, by the oxidation of 4-carbon atom hydrocarbons with molecular oxygen or an oxygen containing gas.

Catalyst precursors of vanadium phosphorus mixed oxide catalysts for hydrocarbon oxidation may be prepared according to methods known in the art.

2

U.S. Patent No. 4,002,650 discloses the preparation of vanadium and phosphorus mixed oxide containing catalysts by reacting vanadium and phosphorus compounds in an aqueous solution, with HCl being utilized as a solvating and reducing agent for vanadium. Similar preparational techniques are described in European Patent Appln. EP—A—3,431 in which the additional step of comminuting the vanadium-phosphorus precursor to a particle size of 500 to 700 microns (0.5 to 0.7 mm) is disclosed.

U.S. Patent No. 4,043,943 discloses the preparation of the catalyst precursor in a liquid organic medium, preferably anhydrous, wherein the vanadium compound is reduced and solvated by gaseous HCl followed by reaction with the phosphorus compound.

The preparation of oxidation catalysts containing the mixed oxides of vanadium and phosphorus is disclosed in U.S. Patent No. 4,244,879 wherein a vanadium compound is at least partially solubilized in an organic liquid medium capable of reducing at least a portion of the vanadium to a +4 valence state, and unsolubilized vanadium having a particle size larger than about 0.1 mm diameter is removed from the medium before addition of a phosphorus-containing compound. The preparation of such catalysts is disclosed in U.S. Patent No. 4,333,853 wherein partial reduction of a pentavalent vanadium compound is effected in the presence of a phosphorus compound in an organic liquid medium capable of reducing the vanadium.

The catalyst precursor may be recovered from the liquid reaction medium in which it was prepared (preferably an essentially anhydrous maintained organic liquid medium) by conventional methods, such as evaporation, filtration, centrifugation or decanting. Preferably, the precursor is dried by heating. Alternatively, the recovered precursor, which is still partially wet with the organic liquid, may be treated with a low boiling solvent such as petroleum ether. In another embodiment, excess preparational reaction media may be substantially removed by vacuum filtration. In yet another embodiment, excess water can be introduced into the precursor containing organic liquid reaction medium, allowing an organic layer to separate from the aqueous layer followed by recovery of the catalyst precursor by drying.

After recovery, the catalyst precursor is subjected to densification and comminution. The order in which the catalyst precursor is densified and comminuted is dependent upon the method used for accomplishing these purposes. For example, the catalyst precursor may be densified by tableting or pelleting the catalyst precursor, and thereafter crushing or grinding the densified material to prepare it for formation of the microspheroidal particles. Alternatively, the catalyst precursor may be recovered by drying or spray drying, and thereafter subjected to dry ball milling in order to both densify the precursor material and comminute the catalyst precursor to an average particle size less than 1 micrometer in diameter. The steps of densifying and comminuting the catalyst precursor may be repeated such that the final fluidizable catalyst particle has a bulk density equal to or greater than 0.75 grams per cubic centimeter, preferably greater than or equal to 1 gram per cubic centimeter.

The densified, comminuted catalyst precursor is then formed into microspheroidal fluidizable particles. Formation may be accomplished by the oil drop method, in which an aqueous solution of the catalyst precursor is dropped into a hot oil bath to cause the formation of spheroidal particles. Preferably, the microspheroidal fluidizable particles are formed by spray drying an aqueous slurry of the catalyst precursor.

The formation of fluidizable particles by crushing and screening to form a fluidizable fraction is not suitable for forming attrition resistant catalysts, as the particles easily abraid during fluid bed operation due primarily to their irregular surface texture. Catalysts formed by crushing and screening also are more prone to fracturing, for the same reason.

If spray drying is to be utilized, the catalyst precursor preferably should be uncalcined when introduced into water to form the aqueous slurry. Substantial contacting of the calcined vanadium phosphorus mixed oxide catalyst with water (at less than 100°C) reduces the activity of the catalyst, particularly if calcined in air.

The solids content of the catalyst precursor containing aqueous slurry should be adjusted to 25 to 60 weight % preferably above 40 weight %. The catalyst precursor-containing aqueous slurry is then spray dried to form uniform, microspheroidal particles having a particle size range of between 20 to 300 micrometers, generally between 20 to 240 micrometers. Spray drying may be accomplished by methods known in the art.

Inert diluents or supports may be added to the fluid bed catalyst by the addition of the diluent or support before or during any of the densification, comminution, and formation of the microspheroidal fluidizable particle steps. Such inert diluents or supports may include silica, alumina, alumina silica, titania, niobia and silicon carbide.

The process of the present invention, however, does not rely solely upon the addition of attrition resistant supports to impart attrition resistance to the catalyst. The particular combination of steps of the present invention results in the formation of an attrition resistant catalyst in which the level of inert supports may be extremely low, or absent. Generally the catalysts of the present invention include at least 70% active material. Preferably, the attrition resistant fluidizable catalyst of the present invention contain at least 80% active material, and most preferably at least 90% active material.

The fluidizable particles prepared above are subjected to calcination under fluidization-type conditions. Fluidization conditions can be determined readily by those of skill in the art, and include the introduction of a gas stream into a catalyst containing fluid bed vessel sufficient to "raise" the catalyst bed and contact substantially all catalyst particles with the gaseous feed, maintaining isothermal temperature control. Other calcination techniques such as cascading calcination, which, similar to fluidization calcination, provide homogeneous gas contacting of the catalyst particles and relatively isothermal temperature control, may be utilized according to the present invention, to result in fluidization-type conditions sufficient to impart attrition resistance to the calcined catalyst. Fluid bed calcination is, however, preferred.

The catalyst is calcined in air or an oxygen containing gas under fluidization-type conditions at a temperature range of 300°C to 450°C. The catalyst may be calcined additionally in the presence of hydrocarbon, an inert gas, steam or both. Preferably, the calcination temperature is increased from 300—325°C steadily to 400—425°C, preferably at a rate of 0.5° to 5°C per minute. Calcination times vary depending upon method of preparation, catalyst composition and amount of catalyst, but generally calcination is conducted for a period of time greater than 1 hour.

The catalyst precursor may contain promoter elements including but not limited to alkali metals, alkaline earth metals, Ti, Cr, W, Ta, U, Co, Mo, Fe, Zn, Hf, Zr, Mn, As, Sb, Te, Bi, Sn, Ge, Nb, Ni, Cu, Cd, Ce, Th, rare earths or mixtures thereof. These may be incorporated into the catalyst precursor in any of the methods known in the art, such as inclusion via the liquid reaction medium prior to or after reduction of the vanadium, or during one or more steps of the preparation of the fluidizable catalyst. The promoter elements may be added to the catalyst as metals, oxides, hydroxides, carbonates, or salts such as halides, nitrates, acetates, formates, butyrates, benzylates, and the like. The molar ratio of promoter elements to vanadium is generally 0.001:1 to 1:1, preferably 0.01:1 to 0.5:1.

Catalysts suitable for the production of maleic anhydride from 4-carbon atom hydrocarbons generally have a phosphorus to vanadium ratio of 2:1 to 0.5:1 preferably 0.8:1 to 1.3:1. Most preferred is a P/V ratio of 1.2:1. These catalysts preferably exhibit an average valence for vanadium within the range of +3.5 to +4.6, preferably approximately +4.

The attrition resistant fluid bed catalyst prepared by the process of the present invention may be utilized in oxidation type fluid bed reactors known in the art.

The hydrocarbon reacted to form maleic anhydride may be n-butane, n-butenes 1,3-butadiene, or a mixture thereof. The use of n-butane or mixture of hydrocarbons that are produced in refinery streams is preferred. The molecular oxygen is most conveniently added as air, but synthetic streams containing molecular oxygen are also suitable. In addition to the hydrocarbon and molecular oxygen, other gases may be added to the reactant feed. For example, steam or nitrogen could be added to the reactants.

The ratio of the reactants may vary widely and are not critical. Preferred oxygen/hydrocarbon ratios in the reactor feed are from 4 to 20 moles of oxygen per mole of hydrocarbon.

The reaction temperature may vary widely and is dependent upon the particular hydrocarbon and catalyst employed. Generally, temperatures of 325°C to 500°C are preferred. The reaction may be conducted at atmospheric, superatmospheric or subatmospheric pressure, although operation at superatmospheric pressure is preferred.

The following Examples illustrate the invention:—

Example 1

A fluidizable catalyst of the formula 90% $V_1P_{1.2}O_x$/10% $SiO_2$ (where x is the number of oxygens required to satisfy the valence requirements of the other elements) was prepared by the following method. The catalyst precursor was prepared by introducing 7.3 parts of $V_2O_5$ and 10.5 parts mixed phosphoric acid into 100 parts isobutanol with stirring, and refluxing the resulting slurry for 6 hours. The mixed phosphoric acid source contained 87% orthophosphoric acid, 12% pyrophosphoric acid and 1% higher phosphoric acids based upon total weight of phosphoric acid. The resulting vanadium phosphorus catalyst precursor precipitate was filtered and dried. The vanadium phosphorus catalyst precursor was then comminuted to an average particle size less than 1 micrometer in diameter and was densified by dry ball milling the precursor.

The comminuted and densified catalyst precursor was introduced into water, and a portion of Nalco Silica Sol (trade designation of Nalco Chemical Co.) was added to the resulting slurry with stirring in an amount necessary to comprise 10% silica by weight based upon total weight of precursor and silica. The solids content of the slurry was above 45% by weight. The resulting slurry was spray dried to yield uniform, microspheroidal catalyst particles. These fluidizable catalyst particles were charged to a fluid bed vessel and under fluidization conditions, were calcined initially to 300—325°C in an air stream. The calcination temperature was then raised at approximately 2°C per minute to 400—425°C, with heating at this level for 1 hour.

The fluidizable, attrition resistant catalysts were utilized in the production of maleic anhydride from n-butane, and were thereafter subjected to the following attrition test. Results of the attrition test are included in Table I below. Microscopic inspection of the attrition tested catalyst particles prepared by the process of the invention revealed very little fragmentation.

Attrition resistance was measured in an apparatus as described in the American Cyanamide Co., "Test Methods for Synthetic Cracking Catalyst", 1957, at page 44. In the test, air is admitted through openings of a perforated plate with sufficient velocity to cause jets of the catalyst to be blown into the main bed of the catalyst at a high velocity. The air velocity through the perforated plate was 1000 feet (305 metres) per second.

4

Fifty grams of the catalyst, screened through 140 on 230 mesh (0.105 mm on 0.063 mm) was charged to the unit and subjected to the air velocity of 1000 feet (305 meters) per second. The attrited material carried over into the collection flask after 5 hours was weighed and calculated as percent loss in 0—5 hours. The procedure was repeated for 15 more hours and the attrited material was collected over the period (from 5 to 20 hours total), and the percent lost in the 5—20 hour period was calculated as the percentage attrition · equal to 100 times the grams recovered in the 5 to 20 hour period divided by the quantity of 50 grams initial charge minus the number of grams recovered in the 0—5 hour period.

Example 2

A fluid bed catalyst having the formula 95% $V_1P_{1.2}O_x$/5% $SiO_2$ was prepared according to the procedure of Example 1, except that only 5% by weight silica was added to the slurry to be spray dried. The resulting fluidizable catalyst was subjected to the calcination procedure described in Example 1. After utilization of the catalyst for the production of maleic anhydride from n-butane, the percent attrition was tested according to the procedure of Example 1. Results of the attrition test are reported in Table 1.

Comparative Example 3

A fluid bed catalyst having the formula 95% $V_1P_{1.2}O_x$/5% $SiO_2$ was prepared according to the procedure of Example 2, except that the fluidizable catalyst particles were subjected to a static calcination (no fluidization) under air. After utilization of the catalyst for the production of maleic anhydride from n-butane, the percent attrition was tested according to the procedure of Example 1. Results of the attrition test are reported in Table 1.

As is reported in the Table, the catalyst which was not subjected to fluidization-type conditions during calcination has much poorer attrition resistance as compared to the fluid bed calcined catalysts.

It is to be noted that the attrition test utilized is an accelerated test, and the attriting forces to which the catalyst is subjected are several hundred times greater than the forces which would be encountered in normal fluid bed operation. In fact, percent attrition was found to fall logarithmically upon reduction of the air velocity. For example, a catalyst was tested which exhibited 8.3% attrition (weight loss) during 0—5 hours at an air velocity of 1000 feet (305 metres) per second, and a catalyst prepared in the same batch was subjected to 500 feet (15.24 metres) per second velocities, exhibiting only a 0.8% weight loss in the 0—5 hour period.

Example 4

A fluid bed catalyst having the formula 100% $V_{1.0}P_{1.2}O_x$ was prepared according to the procedure of Example 1 except that no silica was added to the slurry. During the preparation the catalyst precursor was comminuted and densified by dry ball milling. The spray dried particles were subjected to calcination under fluidization conditions in air, initially at 300°C with heating at approximately 2°C per minute until 425°C was reached, and with calcination continuing at 425°C for 45 minutes.

The fluid bed calcined catalyst was subjected to the attrition test according to the procedure of Example 1 both before and after utilization of the catalyst for the production of maleic anhydride from n-butane, and the results of the tests are reported in Table 1.

Comparative Example 5

A fluid bed catalyst having the formula 100% $V_1P_{1.2}O_x$ was prepared according to the procedure of Example 4, except that the fluidizable particles were subjected to a static calcination (no fluidization) under air for $2\frac{1}{2}$ hours. The calcined catalyst was subjected to the attrition test according to the procedure of Example 1, and test results are reported in Table 1.

The most significant test period is considered to be the 5—20 hour test period. As is demonstrated by the results reported in Table 1, the process of the present invention produces a much more highly attrition resistant catalyst than a procedure which omits fluidization of the catalyst particles during calcination. The catalyst prepared according to the process of the present invention exhibited a much lower percentage weight loss as a result of the attrition test.

Comparative Example 6

A fluid bed catalyst of the formula 100% $V_1P_{1.2}O_x$ was prepared by a procedure which omitted the densification of the catalyst precursor, the comminution step being conducted by the ball milling of an aqueous slurry of the catalyst precursor, preventing the densification.

In addition, the spray dried catalysts were subjected to a static calcination in air, at 400°C for 1 hour. The resulting fluidizable catalyst was subjected to the attrition test according to the procedure of Example 1, and the results of the test are reported in Table 1. As is demonstrated by the results reported in the Table the omission of the densification step and the fluid bed calcination resulted in the majority of the catalyst being attrited away even in the first 5 hour test period.

Comparative Example 7

A fluid bed catalyst of the formula 100% $V_1P_{1.2}O_x$ was prepared by a procedure which omitted the densification of the catalyst precursor, the comminution step being conducted in an air mill (in which dry particles impinge upon themselves and shatter). The spray dried catalysts were additionally subjected to static calcination. The resulting fluidizable catalyst was subjected to the attrition test of Example 1, and as shown in Table 1, the majority of the catalyst was attrited away in the first 5 hour test period.

Example 8

In order to demonstrate the suitability of the attrition resistant fluid bed catalysts prepared by the process of the present invention for the fluid bed production of maleic anhydride, the catalyst prepared in Example 4 was used to produce maleic anhydride from n-butane in a fluid bed reactor consisting of a 61 cm length of stainless steel tubing having an inner diameter of 4.1 cm, having a stainless steel sparger at the bottom of the tube to act as a gas (air) distributor, with an axial 0.64 cm outer diameter thermowell and a separate hydrocarbon inlet at the bottom of the tube. The reactor was fitted with internal gas redistributing baffles. Gas preheating and reactor temperature control was accomplished by placement of the reactor unit in a thermostated fluidized sand bath.

Flasks for receiving the product maleic anhydride were air cooled, and tail gases were routed to a gas chromatograph for analysis. The results are stated in terms as follows:

$$\text{Single Pass Yield} = \frac{\text{Moles of Maleic Anhydride Formed}}{\text{Moles of Butane Fed}} \times 100$$

$$\text{Total Conversion} = \frac{\text{Moles of Butane Reacted}}{\text{Moles of Butane Fed}} \times 100$$

$$\text{Selectivity} = \frac{\text{Single Pass Yield}}{\text{Total Conversion}} \times 100$$

The throughput of hydrocarbon feed in the production of maleic anhydride, or the working rate imposed upon the catalyst is expressed as WWH (weight of feed/weight of catalyst/hour) and was adjusted to 0.050.

The catalyst was initially activated, and at 116 hours of operation exhibited a Total Conversion of 90.1% at a Selectivity to maleic anhydride of 62.2% and a Single Pass Yield of maleic anhydride of 56.1%.

As can be seen from the results reported in Table 1 and the Examples above, fluid bed catalysts containing the mixed oxides of vanadium and phosphorus may be prepared according to the present invention, such catalysts being highly attrition resistant and useful in the production of maleic anhydride from 4-carbon atom hydrocarbons. The fluid bed catalysts the prepared are suitable for use as commercial fluid bed catalysts.

TABLE 1

| Example No. | When tested[a] | % Attrition[b] | |
| | | 0—5 Hours | 5—20 Hours |
| --- | --- | --- | --- |
| 1 | After Use | 5.5 | 10.1 |
| 2 | After Use | 3.3 | 7.5 |
| C3 | After Use | 6.4 | 25.3 |
| 4 | Before Use | 1.4 | 4.1 |
| | After Use | 3.2 | 12.2 |
| C5 | Before Use | 7.2 | 32.1 |
| C6 | Before Use | 53.8 | 10.4 |
| C7 | Before Use | 69.8 | 11.0 |

[a]Before or after use to produce maleic anhydride from n-butane
[b]% weight loss during period of the test.

Claims

1. A process for the preparation of an attrition resistant microsperoidal fluid bed oxidation catalyst containing the mixed oxides of vanadium and phosphorus, comprising
(a) preparing a vanadium phosphorus mixed oxide containing catalyst precursor;

(b) densifying the catalyst precursor;

(c) comminuting the catalyst precursor to an average particle size less than one micrometer in diameter;

(d) forming fluidizable particles from the densified, comminuted catalyst precursor;

(e) calcining the fluidizable particles under fluidization-type conditions.

2. A process as claimed in claim 1 characterised in that the catalyst precursor is prepared in an organic liquid.

3. A process as claimed in claim 2 characterised in that the catalyst precursor is prepared in an organic liquid slurry.

4. A process as claimed in any of claims 1 to 3 characterised in that the substantial portion of said catalyst precursor is comminuted to an average particle size of less than about one-half micrometer average diameter.

5. A process as claimed in any of claims 1 to 4 characterised in that the fluidizable particles are formed by introducing the catalyst precursor into water to form an aqueous slurry and spray drying said slurry to form microspheroidal catalyst particles.

6. A process as claimed in claim 5 characterised in that the aqueous slurry has a solids content of 25 to 60 weight percent.

7. A process as claimed in any of claims 1 to 6 characterised in that a substantial portion of said fluidizable particles have a particle size of less than 300 micrometers.

8. A process as claimed in any of claims 1 to 7 characterised in that the catalyst precursor is densified and comminuted by dry ball milling the dried catalyst precursor.

9. A process as claimed in any of claims 1 to 8 characterised in that the bulk density of said fluidizable catalyst is greater than or equal to 1 gram per cubic centimeter.

10. A process as claimed in any of claims 1 to 9 characterised in that the fluidizable catalyst additionally comprises promoter elements selected from the group consisting of alkali metals, alkaline earth metals, Ti, Cr, W, Ta, U, Co, Mo, Fe, Zn, Hf, Zr, Mn, As, Sb, Te, Bi, Sn, Ge, Nb, Ni, Cu, Cd, Th, Ce, rare earths or mixture thereof.

11. An attrition resistant, fluidizable microspheroidal oxidation catalyst, comprising the mixed oxides of vanadium and phosphorus characterised by an average valence state of vanadium from +3.5 to +4.6 and a phosphorus to vanadium ratio of 0.5:1 to 2:1 wherein said catalyst has been prepared by a process as claimed in any of claims 1 to 10.

12. A process for the production of maleic anhydride by the oxidation of 4 carbon atom hydrocarbons with molecular oxygen or an oxygen containing gas in a fluid bed reactor at a reactor temperature of 325°C to 500°C in the presence of an attrition resistant, fluidizable microspheroidal catalyst containing the mixed oxides of vanadium and phosphorus, wherein the catalyst has been prepared by a process as claimed in any of claims 1 to 10.

**Patentansprüche**

1. Ein Verfahren zur Herstellung eines abnutzungsfesten mikrosphärischen Fließbatt-Oxidationskatalysators, enthaltend die gemischten Oxide von Vanadium und Phosphor, umfassend:

a) die Herstellung eines gemischtes Vanadium-Phosphor-Oxid enthaltenden Katalysatorvorläufers;

b) das Verdichten des Katalysatorvorläufers;

c) die Zerkleinerung des Katalysatorvorläufers auf eine durchschnittliche Teilchengröße von weniger als 1 Mikrometer Durchmesser;

d) die Bildung von verwirbelbaren Teilchen aus dem verdichteten, zerkleinerten Katalysatorvorläufer;

e) das Kalzinieren der verwirbelbaren Teilchen unter Bedingungen vom Verwirbelungs-Typ.

2. Ein Verfahren, wie in Anspruch 1 beansprucht, dadurch gekennzeichnet, daß der Katalysatorvorläufer in einer organischen Flüssigkeit hergestellt wird.

3. Ein Verfahren, wie in Anspruch 2 beansprucht, dadurch gekennzeichnet, daß der Katalysatorvorläufer in einer organischen flüssigen Aufschlämmung hergestellt wird.

4. Ein Verfahren, wie in einem der Ansprüche 1 bis 3 beansprucht, dadurch gekennzeichnet, daß der wesentliche Anteil des Katalysatorvorläufers auf eine durchschnittliche Teilchengröße von weniger als etwa ein halbes Mikrometer durchschnittlichen Durchmesser zerkleinert wird.

5. Ein Verfahren, wie in einem der Ansprüche 1 bis 4 beansprucht, dadurch gekennzeichnet, daß die verwirbelbaren Teilchen gebildet werden durch Einführen des Katalysatorvorläufers in Wasser, zur Bildung einer wässrigen Aufschlämmung, und Sprühtrocknen dieser Aufschlämmung zur Bildung von mikrosphärischen Katalysatorteilchen.

6. Ein Verfahren, wie in Anspruch 5 beansprucht, dadurch gekennzeichnet, daß die wässrige Aufschlämmung einen Feststoffgehalt von 25 bis 60 Gew.-% hat.

7. Ein Verfahren, wir in einem der Ansprüche 1 bis 6 beansprucht, dadurch gekennzeichnet, daß ein wesentlicher Anteil der verwirbelbaren Teilchen eine Teilchengröße von weniger als 300 Mikrometer hat.

8. Ein Verfahren, wie in einem der Ansprüche 1 bis 7 beansprucht, dadurch gekennzeichnet, daß der Katalysatorvorläufer durch Trocken-Kugelvermahlen des getrockneten Katalysatorvorläufers verdichtet und zerkleinert wird.

9. Ein Verfahren, wie in einem der Ansprüche 1 bis 8 beansprucht, dadurch gekennzeichnet, daß die Schüttdichte des verwirbelbaren Katalysators größer als oder gleich wie 1 Gramm pro Kubikmeter ist.

10. Ein Verfahren, wie in einem der Ansprüche 1 bis 9 beansprucht, dadurch gekennzeichnet, daß der verwirbelbare Katalysator außerdem Promotorelemente enthält, ausgewählt aus der Gruppe von Alkalimetallen, Erdalkalimetallen, Ti, Cr, W, Ta, U, Co, Mo, Fe, Zn, Hf, Zr, Mn, As, Sb, Te, Bi, Sn, Ge, Nb, Ni, Cu, Cd, Th, Ce, Seltenen Erden oder Gemischen davon.

11. Ein abnutzungsfester, verwirbelbarer, mikrosphärischer Oxidationskatalysator, umfassend die gemischten Oxide von Vanadium und Phosphor, gekennzeichnet durch einen durchschnittlichen Wertigkeitszustand des Vanadiums von +3,5 bis +4,6 und ein Phosphor-zu-Vanadium-Verhältnis von 0,5:1 bis 2:1, wobei der Katalysator hergestellt wurde durch ein Verfahren, wie in einem der Ansprüche 1 bis 10 beansprucht.

12. Ein Verfahren zur Herstellung von Maleinsäureanhydrid, durch Oxidation von Kohlenwasserstoffen mit 4 Kohlenstoffatomen mit molekularem Sauerstoff oder einem Sauerstoff enthaltenden Gas, in einem Fließbettreaktor, bei einer Reaktortemperatur von 325°C bis 500°C, in Anwesenheit eines abnutzungsfesten, verwirbelbaren mikrospärischen Katalysators, der die gemischten Oxide von Vanadium und Phosphor enthält, wobei der Katalysator hergestellt wurde durch ein Verfahren, wie in einem der Ansprüche 1 bis 10 beansprucht.

## Revendications

1. Procédé pour la préparation d'un catalyseur d'oxydation en lit fluidisé microsphéroïdal résistant à l'attrition contenant les oxydes mixtes de vanadium et de phosphore, consistant:
   (a) à préparer un précurseur de catalyseur contenant un oxyde mixte de vanadium et de phosphore;
   (b) à densifier le précurseur du catalyseur;
   (c) à broyer le précurseur du catalyseur à une taille moyenne de particules inférieure à un micromètre de diamètre;
   (d) à former des particules fluidisables à partir du précurseur du catalyseur densifié, broyé;
   (e) à calciner les particules fluidisables dans des conditions du type fluidisation.

2. Procédé selon la revendication 1, caractérisé par le fait que le précurseur du catalyseur est préparé dans un liquide organique.

3. Procédé selon la revendication 2, caractérisé par le fait que le précurseur du catalyseur est préparé dans une bouillie de liquide organique.

4. Procédé selon l'une quelconque des revendications 1 à 3, caractérisé par le fait qu'une partie notable de ce précurseur du catalyseur est broyée à une taille moyenne de particule inférieure à environ un demi-micromètre de diamètre moyen.

5. Procédé selon l'une quelconque des revendications 1 à 4, caractérisé par le fait que les particules fluidisables sont formées en introduisant le précurseur du catalyseur dans de l'eau pour former une bouillie aqueuse, et en séchant par vaporisation cette bouillie pour former des particules de catalyseur microsphéroïdales.

6. Procédé selon la revendication 5, caractérisé par le fait que la bouillie aqueuse a une teneur en matières solides de 25 à 60 pour cent en poids.

7. Procédé selon l'une quelconque des revendications 1 à 6, caractérisé par le fait qu'une partie notable desdites particules fluidisables ont une taille de particule inférieure à 300 micromètres.

8. Procédé selon l'une quelconque des revendications 1 à 7, caractérisé par le fait que le précurseur du catalyseur est densifié et broyé par broyage dans un broyeur à boulets à sec du précurseur du catalyseur séché.

9. Procédé selon l'une quelconque des revendications 1 à 8, caractérisé par le fait que la densité apparente dudit catalyseur fluidisable est supérieure ou égale à 1 gramme par centimètre cube.

10. Procédé selon l'une quelconque des revendications 1 à 9, caractérisé par le fait que le catalyseur fluidisable comprend en outre des éléments promoteurs choisis dans le groupe constitué des métaux alcalins, des métaux alcalinoterreux, de Ti, Cr, W, Ta, U, Co, Mo, Fe, Zn, Hf, Zr, Mn, As, Sb, Te, Bi, Sn, Ge, Nb, Ni, Cu, Cd, Th, Ce des terres rares et des mélanges de ceux-ci.

11. Catalyseur d'oxydation microsphéroïdal fluidisable, résistant à l'usure, comprenant les oxydes mixtes de vanadium et de phosphore, caractérisé par un état de valence moyen du vanadium de +3,5 à +4,6 et par un rapport du phosphore au vanadium de 0,5:1 à 2:1, dans lequel ledit catalyseur a été préparé par un procédé selon l'une quelconque des revendications 1 à 10.

12. Procédé pour la production d'anhydride maléique par oxydation d'hydrocarbures en $C_4$ avec de l'oxygène moléculaire ou un gaz contenant de l'oxygène dans un réacteur à lit fluidisé à une température de réacteur de 325°C à 500°C en présence d'un catalyseur microsphéroïdal fluidisable résistant à l'usure par attrition contenant les oxydes mixtes de vanadium et de phosphore, dans lequel le catalyseur a été préparé par un procédé selon l'une quelconque des revendications 1 à 10.